# EUROPEAN PATENT SPECIFICATION

(11) **EP 1 220 887 B1**
(45) Date of publication and mention of the grant of the patent: **07.12.2005**
(21) Application number: 00968909.2
(22) Date of filing: 10.10.2000
(51) Int. Cl.: C11D 17/00, C11D 3/38, C11D 3/22, C11D 3/12, C11D 3/386, C12N 9/98

(54) **PROTEIN-CONTAINING GRANULES AND GRANULE FORMULATIONS**
PROTEINHALTIGE KÖRNCHEN UND GRANULATFORMULIERUNGEN
GRANULE CONTENANT DES PROTEINES ET FORMULATIONS DE GRANULES

(30) Priority: 15.10.1999 US 419391
(43) Date of publication of application: 10.07.2002
(73) Proprietor: GENENCOR INTERNATIONAL, INC., Palo Alto, California 94304 (US)
(72) Inventor: GHANI, Mahmood, M., Milpitas, CA 95035 (US)
(74) Representative: Wibbelmann, Jobst
(86) International application number: PCT/US2000/027888
(87) International publication number: WO 2001/029170

(56) References cited:
- EP-A- 0 277 532
- EP-A- 0 304 331
- EP-A- 0 415 652
- WO-A-89/08694
- WO-A-97/39116
- WO-A-99/32613
- US-A- 5 739 091

## Description

### Field of the Invention

This invention relates to protein-carrying granules, e.g., enzyme granules, and compositions for use therein, as well as processes for producing such granules.

### Background of the Invention

The use of proteins such as pharmaceutically important proteins, e.g., hormones, and industrially important proteins, e.g., enzymes, has been rapidly growing in recent years. Today, for example, enzymes find frequent use in the starch, dairy, and detergent industries, among others.

In the detergent industry, in particular, enzymes are often configured in a granular form, with an eye toward achieving one or more desirable storage and/or performance characteristics, depending upon the particular application at hand. In these regards, the industry has offered numerous developments in the granulation and coating of enzymes, several of which are exemplified in the following patents and publications:

U.S. Patent 4,106,991 describes an improved formulation of enzyme granules by including within the composition undergoing granulation, finely divided cellulose fibers in an amount of 2-40% w/w based on the dry weight of the whole composition. In addition, this patent describes that waxy substances can be used to coat the particles of the granulate.

U.S. Patent 4,689,297 describes enzyme containing particles which comprise a particulate, water dispersible core which is 150 - 2,000 microns in its longest dimension, a uniform layer of enzyme around the core particle which amounts to 10%-35% by weight of the weight of the core particle, and a layer of macro-molecular, film-forming, water soluble or dispersible coating agent uniformly surrounding the enzyme layer wherein the combination of enzyme and coating agent is from 25-55% of the weight of the core particle. The core material described in this patent includes clay, a sugar crystal enclosed in layers of corn starch which is coated with a layer of dextrin, agglomerated potato starch, particulate salt, agglomerated trisodium citrate, pan crystallized NaCl flakes, bentonite granules or prills, granules containing bentonite, kaolin and diatomaceous earth or sodium citrate crystals. The film forming material may be a fatty acid ester, an alkoxylated alcohol, a polyvinyl alcohol or an ethoxylated alkylphenol.

U.S. Patent 4,740,469 describes an enzyme granular composition consisting essentially of from 1-35% by weight of an enzyme and from 0.5-30% by weight of a synthetic fibrous material having an average length of from 100-500 micron and a fineness in the range of from 0.05-0.7 denier, with the balance being an extender or filler. The granular composition may further comprise a molten waxy material, such as polyethylene glycol, and optionally a colorant such as titanium dioxide.

U.S. Patent 5,324,649 describes enzyme-containing granules having a core, an enzyme layer and an outer coating layer. The enzyme layer and, optionally, the core and outer coating layer contain a vinyl polymer.

WO 91/09941 describes an enzyme containing preparation whereby at least 50% of the enzymatic activity is present in the preparation as enzyme crystals. The preparation can be either a slurry or a granulate.

WO 97/12958 discloses a microgranular enzyme composition. The granules are made by fluid-bed agglomeration which results in granules with numerous carrier or seed particles coated with enzyme and bound together by a binder.

WO 99/32613 teaches a granular composition including a core comprised of a protein or enzyme mixed with a sugar or sugar alcohol and a structuring agent, such as a polysaccharide or polypeptide. The granular composition can further include a barrier layer formed over the core, as well as an outside coating material. The barrier layer can be comprised of, for example, inorganic salts or organic acids or salts. Exemplary coating materials include, for example, water soluble or dispersible film-forming materials, such as polyvinyl alcohol (PVA), polyvinyl pyrrolidone (PVP), or cellulose derivatives such as methylcellulose. Typically, the outer coating further includes a pigment, such as TiO₂, to impart a desired color to the composition.

Notwithstanding such developments, there is a continuing need for enzyme granules which have additional beneficial or improved characteristics. One such characteristic needed in the enzyme granulation industry are granule formulations having good stability, particularly at high humidity and temperature. Especially desirable, are enzyme granules with good stability during storage in, for example, bleach-containing detergent formulas, such as those containing peroxygen bleaches, e.g., sodium perborate or sodium percarbonate.

It is an object of the present invention to provide compositions which can be used as layers or coatings surrounding enzyme-containing regions, e.g., cores, of granules, resulting in granules having good storage stability. An additional object of the present invention is to provide granular formulations including such compositions, e.g., as one or more layers or coatings. It is yet a further object hereof to provide a method of making enzyme granules having good storage stability.

### Summary of the Invention

One aspect of the present invention provides a granule comprising an interior, protein-containing region (e.g., a core), a matrix layer surrounding the protein-containing region, the matrix layer comprising a mixture that includes a finely divided drying agent, polysaccharide, and sugar or sugar alcohol, and an outer layer of a film-forming polymer, the outer layer being substantially free of pigments to form a clear coating. Optionally, the matrix can further include a surfactant or wetting agent, e.g., Neodol. According to one embodiment, the matrix layer is substantially free of enzymes.

In one embodiment, the granule is characterized by a low affinity for moisture. For example, the granule in this embodiment, when exposed at 50 degrees C and 70% relative humidity, will pick up less than 10%, and preferably less than 5%, moisture weight gain "at equilibrium", i.e., when the moisture pick-up has generally leveled out. Most preferably, the moisture weight gain levels out at about 2 to 3%.

The drying agent of the matrix can be, for example, substantially water-insoluble, water-dispersible particles having a mean diameter of no greater than about 30 micrometers. In one embodiment, the drying agent is comprised at least in part of a pigment or clay. Exemplary drying agents include TiO₂, talc, calcium carbonate, bentonite, diatomaceous earth, and any mixture thereof. In one particularly preferred embodiment, the drying agent is TiO₂.

Exemplary polysaccharides include starch, carrageenan, cellulose, gum arabic, acacia gum, xanthan gum, locust bean gum, and guar gum, and any mixtures thereof. In one preferred embodiment, the polysaccharide is a substantially water-insoluble polysaccharide, e.g., starch.

Exemplary sugars and sugar alcohols include glucose, fructose, raffinose, maltose, lactose, trehalose and sucrose, mannitol, sorbitol, inositol, and any mixtures thereof. In one particularly preferred embodiment, the sugar is sucrose.

According to one embodiment, the protein-containing region (e.g., core) includes at least one enzyme therein. Preferably, substantially all of the enzyme included in the granule, in this embodiment, is contained within the protein-containing region. Any suitable enzymes can be employed, such as proteases, amylases, lipases and/or cellulases.

In another of its aspects, the present invention provides an enzyme granule comprising: (i) an internal, enzyme-containing region; (ii) a matrix layer surrounding the enzyme-containing region, with the matrix layer comprising a mixture that includes (a) a pigment, (b) a sugar or sugar alcohol, and (c) a polysaccharide; and (iii) an outer, substantially pigment-free, polymeric coating surrounding the matrix layer. Optionally, the matrix further can further include a surfactant or wetting agent (e.g., Neodol).

In one preferred embodiment, the granule is substantially free of layers having a high salt content.

Yet a further aspect of the present invention provides a method for making a granule, such as described herein. In one embodiment, the method includes the steps of (i) providing a protein-carrying particle; (ii) forming a barrie matrix layer about the particle by applying a mixture that includes (a) a finely divided drying agent, (b) a polysaccharide, and (c) sugar or sugar alcohol, and (iii) applying a coating about the matrix layer of a film-forming polymer, the outer layer being substantially free of pigments.

Exemplary matrix components include: (i) a pigment or clay as the drying agent, (ii) a sugar or sugar alcohol, and (iii) a polysaccharide. For example, the drying agent can be TiO₂, the sugar can be sucrose, and the polysaccharide can be starch.

According to one embodiment, the coating is formed of a substantially pigment-free, polymeric material (e.g., HPMC).

In an exemplary process, the protein-carrying particle is formed by applying an enzyme mixed together with at least one binder selected from the group consisting of carbohydrates, carbohydrate derivatives, salts, and any mixtures thereof, onto an inert particulate material. In another exemplary process, the protein-carrying particle is formed by layering an enzyme-containing material over a core material.

According to one embodiment, the method includes the step of surrounding the enzyme particulate with a barrier matrix. The barrier matrix, in this embodiment, comprises a mixture that includes a finely divided drying agent, a polysaccharide, and a sugar a sugar alcohol.

Exemplary barrier matrix components include: (i) a pigment or clay as the drying agent, (ii) a sugar or sugar alcohol, and (iii) a polysaccharide. For example, the drying agent can be TiO₂, the sugar can be sucrose, and the polysaccharide can be starch.

The method includes the additional step of applying a substantially pigment-free polymeric coating (e.g., HPMC) about the barrier matrix.

The granules disclosed herein are particularly well suited for use in detergent formulations, such as laundry and/or dish detergents.

The other features, aspects and advantages of the present invention will become apparent from the following detailed description, in conjunction with the appended claims.

### Detailed Description of the Invention

One aspect of the present invention provides a composition suitable for use in connection with protein-carrying granules, e.g., enzyme granules. Generally, the composition comprises a mixture including a drying agent, a polysaccharide, and a sugar or sugar alcohol; together defining a matrix. The matrix is applicable as a coating or layer about a protein-containing region of a granule. For example, the matrix can surround an enzyme-carrying core. Preferably, the matrix is designed to act as a barrier, or physical divider, that slows or prevents the diffusion of substances, such as moisture and bleach, that can adversely effect the protein or enzyme in the protein-containing region of the granule. Accordingly, the matrix is generally referred to herein as a "barrier matrix."

The invention additionally provides a polymeric coating or layer for use in a protein-containing granule. The coating is substantially free of pigments. Coatings formulated in accordance with the invention are generally referred to herein as "clear coatings." It should be noted, in this regard, that the term "clear," in the context of the wording "clear coating," is intended to indicate that the coating is free of pigments, and not necessarily that the coating will allow light to pass through. Thus, it should be understood that the wording "clear coating" encompasses transparent coatings, as well as semitransparent and opaque coatings. The key feature here being that the coating is substantially free of pigments.

As discussed more fully below, the matrix and coating of the present invention is used in combination in an enzyme granule formulation, with the resultant granules exhibiting good stability when challenged with certain destabilizing factors and/or events. Details of the matrix and coating are described next.

As previously mentioned, the barrier matrix of the present invention comprises a mixture of components, including a drying agent, a polysaccharide, and a sugar or sugar alcohol.

Drying agents, contemplated for use in the barrier matrix of the invention, include substantially water-insoluble, water-dispersible materials. Preferably, the drying agent comprises an inorganic material in a finely-divided form, e.g., a particulate material having a mean diameter of no greater than about 30 micrometers, and preferably no greater than about 10 micrometers. In one such embodiment, the drying material has a mean diameter of between about 1-5 micrometers.

Exemplary drying agents include pigments and clays. For example, the drying agent can comprise one or more of the following materials: TiO₂, talc, calcium carbonate, bentonite, diatomaceous earth, and any mixture thereof. In one particularly preferred embodiment, the drying agent is TiO₂.

Exemplary sugars and sugar alcohols include glucose, fructose, raffinose, maltose, lactose, trehalose, sucrose, mannitol, sorbitol, inositol, and any mixtures thereof. In one particularly preferred embodiment, the sugar agent is sucrose.

Exemplary polysaccharides include starch, carrageenan, cellulose, gum arabic, acacia gum, xanthan gum, locust bean gum, and guar gum, and any mixtures thereof. In a particularly preferred embodiment, the polysaccharide is a substantially water-insoluble polysaccharide, such as starch (e.g., unmodified, raw or non-hydrated starch).

With further regard to the polysaccharide, it should be noted that polysaccharides typically have the simultaneous desirable properties of high molecular weight and high water solubility. Without wishing to be bound by theory, it is believed that the high molecular weight of the polysaccharide contributes two important properties which a sugar or sugar alcohol matrix alone would lack: (1) providing cohesion and strength to the particle, greatly reducing the tendency of the particle to dust; and (2) serving as a diffusion barrier to water and small molecules by virtue of forming a polymer network or "cage" throughout the matrix structure. This greatly improves the stability of the granule.

The particular polysaccharides also typically have an anti-tack characteristic which is helpful in reducing the binder characteristic of the sugar or sugar alcohol, and allowing matrix layers to be built up―for example in fluid-bed coating―at rapid rates without agglomeration.

Sugars and sugar alcohols and polysaccharide also have high water solubility or dispersibility. A matrix formula can be easily prepared which includes a sugar or sugar alcohol, a polysaccharide, and a drying agent as a solution or slurry with high total solids concentration. Total solution or slurry solids concentrations of 20-50% w/w or more can be formulated. These concentrated mixtures are highly desirable in that they can be formed into granules with a minimal need for evaporating water, an advantage in any granulation and drying process.

Optionally, the matrix of the present invention can further include a surfactant or wetting agent. The surfactant or wetting agent can be useful, for example, to help keep the drying agent of the maxtix, e.g., TiO₂, in suspension. Exemplary materials that can be incorporated into the matrix include Neodol, tallow alcohols, fatty acids, fatty acid salts such as magnesium stearate and fatty acid esters. In one particularly preferred embodiment, Neodol is employed in the matrix.

Details of the polymeric coating or layer of the present invention will now be described. The coating is comprised of a film-forming polymer, such as polyvinyl alcohol, polyvinyl pyrrolidone, cellulose derivatives such as methylcellulose, hydroxypropyl methylcellulose, hydroxycellulose, ethylcellulose, carboxymethyl cellulose, hydroxypropyl cellulose, polyethylene glycol, polyethylene oxide, chitosan, gum arabic, xanthan, carrageenan, or any mixtures thereof. As previously mentioned, the polymeric coating is substantially free of pigments and the like. In one particularly preferred embodiment, a clear coating is formed of hydroxypropyl methylcellulose (HPMC), or a combination of HPMC and other polymers. For example, Methocel E15, a hydroxypropyl methylcellulose, is commercially available from Dow Chemical Co., Midland Mich. Optionally, the clear coating can include an agent for raising the gellation temperature, such as one or more sugars (e.g., sucrose), and a plasticizer. Suitable plasticizers include, for example, polyols such as sugars, sugar alcohols, or polyethylene glycols (PEGs), urea, glycol, propylene glycol or other known plasticizers such as triethyl citrate, dibutyl or dimethyl phthalate or water. In one preferred embodiment, the plasticizer is polyethylene glycol (e.g., PEG 400 and/or PEG 600).

As discussed above, the barrier matrix and clear coating of the present invention are used in combination in an enzyme granule formulation. In one preferred arrangement, the clear coating is disposed directly adjacent to the barrier matrix, with the coating being the outermost of the two. It should be noted, however, that one or more intervening layers (i.e., between the matrix and coating) can be included, as well.

A wide variety of protein or enzyme granules can incorporate the matrix and/or coating of the present invention. For example, the barrier matrix and/or clear coating can be embodied in prills, marumes, extrudates, fluidized bed layered granules (e.g., Enzoguard® granules; Genencor International, Inc.), and/or high shear matrix granules (e.g., T-granulates; Novo-Nordisk), among others. In a typical formulation, the granule includes at least one region containing one or more proteins or enzymes about which the matrix can be applied. The protein-containing region can comprise, for example, a core having one or more proteins or enzymes embedded or dispersed therein, and/or coated or layered thereon. Alternatively, or in addition, the protein or enzyme containing region can include an inert particle upon which a protein or enzyme matrix is layered. Or, the protein or enzyme containing region can be substantially homogenous in nature, e.g., a homogenous core. Exemplary granules which can be modified to incorporate the barrier matrix and/or clear coating of the present invention are disclosed in PCT Publication Nos. WO 99/32613, WO 99/32595, and WO 99/32612; as well as in U. S. Pat. Nos. 5,814,501, 5,324,649, 4,689,297, and 4,106,991.

Proteins that are within the scope of the present invention include pharmaceutically important proteins such as hormones or other therapeutic proteins, and industrially important proteins such as enzymes.

Any enzyme or combination of enzymes can be used in the present invention. Preferred enzymes include those enzymes capable of hydrolyzing substrates, e.g. stains. Such enzymes, which are known as hydrolases, include, but are not limited to, proteases (bacterial, fungal, acid, neutral or alkaline), amylases (alpha or beta), lipases, cellulases and mixtures thereof. Particularly preferred enzymes are subtilisins and cellulases. Most preferred are subtilisins such as described in U.S. Patent 4,760,025, EP Patent 130 756 B1 and PCT Application WO 91/06637, and cellulases such as Multifect L250™ and Puradax™, commercially available from Genencor International. Other enzymes that can be used in the present invention include oxidases, transferases, dehydratases, reductases, hemicellulases and isomerases.

The granules of the present invention can also comprise one or more additional coatings or layers, e.g., one or more intermediate layers and/or an overcoating. These can serve any of a number of functions in a granular composition, depending on the end use of the granule. For example, coatings may render an enzyme resistant to oxidation by bleach, bring about desirable rates of dissolution upon introduction of the granule into an aqueous medium, and/or reduce the chances of microbial growth within the granule.

The granules described herein may be made by methods known to those skilled in the art of enzyme granulation, including pan-coating, fluid-bed coating, prilling, disc granulation, spray drying, extrusion, centrifugal extrusion, spheronization, drum granulation, high shear agglomeration, or combinations of these techniques.

One preferred process, contemplated by the present invention, is a fluid-bed spray process. In an exemplary embodiment, the process involves charging fluidizable particles, such as sugar crystals, into a fluid-bed spray apparatus, such as a Vector FL1 fluid bed coater. With the particles fluidized, a first spray can be applied, preferably comprising an aqueous protein or enzyme solution. The first spray can be, for example, a fluid concentrate including a protease, a dissolved sugar and suspended starch. In this way, a first layer or coating can be formed over the fluidized particles. Next, a second spray can be applied, preferably comprising the barrier matrix of the present invention. The second spray can be, for example, a fluidic mixture including a dissolved sugar in water and suspended starch and titanium dioxide. This suspension can also contain a surfactant/wetting agent, such as Neodol, to help keep titanium dioxide in suspension. Next, a third spray can be applied, preferably forming a clear coating about the barrier matrix. The third spray can be, for example, a solution including HPMC (or a combination of HPMC and other polymers) and, optionally, a sugar (e.g., sucrose) for raising the gellation temperature, and/or a plastisizer, such as PEG 400 or PEG 600.

One particularly preferred granule, which can be made as just described, consists essentially of (i) a core comprised of a sugar crystal having an enzyme matrix layered thereover; (ii) a barrier matrix layered over the enzyme matrix; and (iii) a clear coating formed over the barrier matrix. Without committing to any particular theory, it is believed that the improved stability of such a granule is due to the presence, in the barrier matrix layer, of a pigment (e.g., titanium dioxide) and non-hydrated starch, which act as drying agents and shield the enzyme from moisture. Further, the clear coating is believed to provide a substantially continuous film-like overcoat. The known granulated enzyme products typically have the pigment in the outer coat, thereby creating an overcoat with a weaker structure.

Preferred granules, constructed in accordance with the present invention, are characterized by a low affinity for moisture. Thus, such granules are substantially free of layers or coatings having a high affinity for moisture (e.g., salt layers). As used herein, "low affinity for moisture" means that a granule exposed at 50 degrees C and 70% relative humidity will pick up less than 10%, and preferably less than 5%, moisture weight gain "at equilibrium", i.e., when the moisture pick-up has generally leveled out. As can be seen from the results of Example 5 (below), exemplary granule formulations incorporating the matrix of the present invention generally level out at about 2 to 3% moisture weight gain. It is believed that, as a result of such low affinity for moisture, the undesirable intrusion of moisture and associated inactivating substances into the protein-containing region of such granules is significantly reduced. When the barrier matrix and/or clear coating of the invention are embodied in an enzyme granule, the enzymes surrounded thereby typically exhibit very good stability.

The following examples are representative and not intended to be limiting.

### EXAMPLES

### Example 1:

### Enzyme Granule Formulation

An exemplary formulation for a batch of granules, produced using a fluid-bed spray process, is shown below in Table I. The initial spray in this example was applied to 540.0 gms of sucrose crystals charged into a fluid-bed chamber, and suspended therein. The enzyme used was Purafect OxP (Genencor International, Inc.). In this and the following examples, "spray 1" denotes an enzyme matrix formed on a fluidizable particle, "spray 2" denotes the barrier matrix of the present invention, and "spray 3" denotes the clear coating of the present invention. Certain details of the fluid-bed process were substantially as described in Example 2 of WO 99/32613.

**Table I**

| | | |
|---|---|---|
| **SPRAY 1:** | OxP conc. 14.3%solids | 1542.0 gms |
| | Sucrose | 393.0 gms |
| | Starch | 393.0 gms |
| | | |
| **SPRAY 2:** | Corn starch | 224.0 gms |
| | Sucrose | 224.0 gms |
| | TiO2 | 125.0 gms |
| | Neodol | 28.0 gms |
| | | |
| **SPRAY 3:** | Methocel E15 | 56.0 gms |
| | PEG 400 | 8.0 gms |

### Example 2

### Enzyme Granule Formulations

Several additional exemplary formulations for enzyme granules, also produced using a fluid-bed spray process, are shown below in Tables 2, 3, and 4. As with the previous example, the initial spray for each of the following formulations was applied to 540.0 gms of sucrose crystals charged into a fluid-bed chamber, and suspended therein. The enzyme used in these formulations was Properase (Genencor International, Inc.).

**Table 2 -**

| **"Lot 80"** | | |
|---|---|---|
| **SPRAY 1:** | Properase conc. (17.3% solids) | 1200.0 ml |
| | Sucrose | 396.0 gms |
| | Starch | 396.0 gms |
| | | |
| **SPRAY 2:** | Corn starch | 224.0 gms |
| | Sucrose | 224.0 gms |
| | TiO2 | 125.0 gms |
| | Neodol | 28.0 gms |
| | | |
| **SPRAY 3:** | HPMC (Methocel E15) | 48.0 gms |
| | PEG 600 | 8.0 gms |

**Table 3 -**

| **"Lot 81"** | | |
|---|---|---|
| **SPRAY 1:** | Properase conc. (17.3% solids) | 1200.0 ml |
| | Sucrose | 396.0 gms |
| | Starch | 396.0 gms |
| | | |
| **SPRAY 2:** | Corn starch | 224.0 gms |
| | Sucrose | 224.0 gms |
| | TiO2 | 125.0 gms |
| | Neodol | 28.0 gms |
| | | |
| **SPRAY 3:** | HPMC (Methocel E15) | 24.0 gms |
| | Modified starch (Pure Cote (50/50)) | 24.0 gms |
| | PEG 600 | 8.0 gms |

**Table 4 -**

| **"Lot 82"** | | |
|---|---|---|
| **SPRAY 1:** | Properase conc. (17.3% solids) | 1200.0 ml |
| | Sucrose | 396.0 gms |
| | Starch | 396.0 gms |
| | | |
| **SPRAY 2:** | Corn starch | 224.0 gms |
| | Sucrose | 224.0 gms |
| | TiO2 | 125.0 gms |
| | Neodol | 28.0 gms |
| | | |
| **SPRAY 3:** | HPMC (Methocel E15) | 48.0 gms |
| | Sucrose | 8.0 gms |
| | PEG 600 | 8.0 gms |

### Example 3:

### Accelerated Stability Tests using a Laundry Detergent Base

The granules of Example 2 (Lots 80, 81, and 82) were analyzed to determine their stability in three day stressed stability tests. The methods for these procedures were substantially as described in Example 3 of WO 99/32613. In this example, however, a proprietary laundry detergent base was used.

As discussed in WO 99/32613, the accelerated stability test is designed to aid in the development and screening of granular formulations, as it provides an accelerated means of determining relative granule stability. The conditions of the accelerated stability test (AST) are far more severe than enzyme granules or detergents would encounter in realistic storage or transport. The AST is a "stress test" designed to discriminate differences between formulations which would otherwise not be evident for weeks or months.

The AST results set out in Table 5, below, show the percent activity remaining for each of Lots 80, 81, and 82, over a three day period.

**Table 5 -**

| **Percent Activity of the Original** | | | |
|---|---|---|---|
| | **Day 0** | **Day 1** | **Day 3** |
| **Lot 80** | 100.0 | 88.8 | 49.7 |
| **Lot 81** | 100.0 | 91.4 | 41.0 |
| **Lot 82** | 100.0 | 85.4 | 39.7 |
| **74-A** | 100.0 | 89.7 | 32.0 |
| **87-B** | 100.0 | 75.5 | 27.0 |

Stability results for two additional Lots, denoted as 74-A and 87-B, are shown in Table 5, as well. These additional lots have the general formulations shown in Table 6, below. It should be noted that these additional formulations were prepared in a fluid-bed spray apparatus, as above, by applying the sprays to sucrose crystals fluidized in a chamber of the apparatus.

**Table 6**

| | **74-A** | **87-B** |
|---|---|---|
| **SPRAY 1:** | Properase conc. (17.3% solids) | Properase conc. (17.3% solids) |
| | Sucrose | Sucrose |
| | Starch | Starch |
| | | |
| **SPRAY 2:** | Corn starch | None |
| | Sucrose | |
| | TiO2 | |
| | Neodol | |
| | | |
| **SPRAY 3:** | None | HPMC (Methocel E15) |
| | | Sucrose (optional) |
| | | PEG 600 |

As can be seen from Table 6, Lots 74-A and 87-B are comprised of granules, much like those set forth in Examples 1 and 2, above, except that the granules of Lot 74-A lack a clear coating, and the granules of Lot 87-B lack a barrier matrix layer.

While granules having an enzyme core surrounded only by the matrix barrier (e.g., Lot 74-A) or the clear coating (e.g., Lot 87-B) of the present invention show good stability in the accelerated stability tests (see Table 5, above), the granules including both such layers about an enzyme core (e.g., Lots 80, 81, and 82) exhibit even better stability.

### Example 4:

### Accelerated Stability Tests using an Automatic Dish Detergent Base

The granules of Example 2 (Lots 80, 81, and 82) were further analyzed in three day stressed stability tests to determine stability in a proprietary automatic dish detergent base. As with the previous example, the methods for these procedures were substantially as described in Example 3 of WO 99/32613. Here, however, the studies were carried out at 40°C and 70% relative humidity.

Table 7, below, shows the percent activity remaining for each of Lots 80, 81, and 82, over a three day period. Results for a commercially available protease granule under the same conditions are shown, as well.

**Table 7 -**

| **Percent Activity of the Original** | | | | |
|---|---|---|---|---|
| | **Day 0** | **Day 1** | **Day 2** | **Day 3** |
| **Lot 80** | 100.0 | 87 | 51 | 32 |
| **Lot 81** | 100.0 | 71 | 44 | 27 |
| **Lot 82** | 100.0 | 86 | 51 | 37 |
| **Commercial Protease Granule** | 100.0 | 55 | 25 | 19 |

### Example 5:

### Accelerated Stability Tests using an Automatic Dish Detergent Base

As previously discussed, preferred granules of the present invention have a low affinity for moisture. Thus, such granules (e.g., the granules of Lots 80, 81, 82, 74-A, 87-B) are substantially free of layers or coatings having a high affinity for moisture (e.g., salt layers). As used herein, "low affinity for moisture" means that a granule exposed at 50 degrees C and 70% relative humidity will pick up less than 10%, and preferably less than 5%, moisture weight gain "at equilibrium", i.e., when the moisture pick-up has generally leveled out. As can be seen from the data of Table 8, below, the exemplary granule formulation of Lot 81 generally leveled out to a moisture weight gain of less than about a 2 to 3%. The commercial protease granule, on the other hand, showed an equilibrium moisture weight gain of at least 17%.

**Table 8 -**

| **Percent weight Gain** | | | |
|---|---|---|---|
| | **Day 0** | **Day 5** | **Day 12** |
| **Lot 81 Granule** | 100 | 101.631 | 101.4609 |
| **Commercial Protease Granule** | 100 | 107.0899 | 117.2339 |

## Claims

1. A granule comprising
an interior, protein-containing region;
a barrier matrix layer surrounding said protein-containing region, and comprising a finely divided drying agent, polysaccharide and sugar or sugar alcohol; and
an outer layer of a film-forming polymer surrounding said barrier matrix layer, the outer layer being substantially free of pigments to form a clear coating.

2. The granule of claim 1, wherein the barrier matrix layer further comprises a surfactant or wetting agent.

3. The granule of claim 1, wherein said drying agent is comprised of substantially water-insoluble, water-dispersible particles having a mean diameter of no greater than about 30 micrometers.

4. The granule of claim 1, wherein said drying agent is comprised at least in part of a pigment or clay.

5. The granule of claim 4, wherein said drying agent is selected from the group consisting of TiO₂, talc, calcium carbonate, bentonite, diatomaceous earth, and any mixture thereof.

6. The granule of claim 1, wherein the polysaccharide is selected from the group consisting of starch, carrageenan, cellulose, gum arabic, acacia gum, xanthan gum, locust bean gum, and guar gum, and any mixture thereof.

7. The granule of claim 1, wherein said sugar or sugar alcohol is selected from the group consisting of glucose, fructose, raffinose, maltose, lactose, trehalose and sucrose, mannitol, sorbitol, inositol, and any mixtures thereof.

8. The granule of claim 2, wherein said surfactant or wetting agent is Neodol.

9. The granule of claim 1, wherein the protein is an enzyme selected from the group consisting of protease, amylase, lipase and cellulase.

10. A method for making a granule according to claim 1, comprising:
(i) providing a protein-carrying particle,
(ii) forming a barrier matrix layer about the particle by applying a mixture that includes a finely divided drying agent, polysaccharide and sugar or sugar alcohol, and
(iii) applying a clear coating about said barrier matrix layer of a film-forming polymer, the outer layer being substantially free of pigments.

## Patentansprüche

1. Granalie, umfassend
einen inneren Protein-haltigen Bereich;
eine Barriere-Matrixschicht, die den Protein-haltigen Bereich umgibt und ein fein zerteiltes Trocknungsmittel, Polysaccharid und Zucker oder Zuckeralkohol umfasst; und
eine äußere Schicht aus einem filmbildenden Polymer, welche die Barriere-Matrixschicht umgibt, wobei die äußere Schicht im Wesentlichen frei von Pigmenten ist, so dass ein klarer Überzug gebildet wird.

2. Granalie nach Anspruch 1, in der die Barriere-Matrixschicht weiter ein Tensid oder Benetzungsmittel umfasst.

3. Granalie nach Anspruch 1, in der das Trocknungsmittel in Wasser im Wesentlichen unlösliche, in Wasser dispergierbare Teilchen mit einem mittleren Durchmesser von nicht mehr als etwa 30 Mikrometer umfasst.

4. Granalie nach Anspruch 1, in der das Trocknungsmittel mindestens teilweise aus einem Pigment oder Ton besteht.

5. Granalie nach Anspruch 4, in der das Trocknungsmittel ausgewählt ist aus der Gruppe bestehend aus TiO₂, Talkum, Calciumcarbonat, Bentonit, Diatomeenerde und jeder beliebigen Mischung derselben.

6. Granalie nach Anspruch 1, in der das Polysaccharid ausgewählt ist aus der Gruppe bestehend aus Stärke, Carrageenan, Cellulose, Gummi arabicum, Akaziengummi, Xanthangummi, Johannisbrotgummi und Guar Gum und jeder beliebigen Mischung derselben.

7. Granalie nach Anspruch 1, in der der Zucker oder Zuckeralkohol ausgewählt ist aus der Gruppe bestehend aus Glucose, Fructose, Raffinose, Maltose, Lactose, Trehalose und Saccharose, Mannit, Sorbit, Inosit und jeder beliebigen Mischung derselben.

8. Granalie nach Anspruch 2, in der das Tensid oder Benetzungsmittel Neodol ist.

9. Granalie nach Anspruch 1, in der das Protein ein Enzym ist, das ausgewählt ist aus der Gruppe bestehend aus Protease, Amylase, Lipase und Cellulase.

10. Verfahren zur Herstellung einer Granalie nach Anspruch 1, umfassend:
(i) Bereitstellen eines Protein-tragenden Teilchens,
(ii) Bilden einer Barriere-Matrixschicht um das Teilchen herum durch Auftragen einer Mischung, die ein fein zerteiltes Trocknungsmittel, Polysaccharid und Zucker oder Zuckeralkohol einschließt, und
(iii) Aufbringen eines klaren Überzugs aus einem filmbildenden Polymer um die Barriere-Matrixschicht herum, wobei die äußere Schicht im Wesentlichen frei von Pigmenten ist.

## Revendications

1. Granule comprenant
une région interne contenant des protéines ;
une couche de matrice formant barrière entourant ladite région contenant des protéines, et comprenant un agent desséchant, un polysaccharide et un sucre ou un alcool de sucre finement divisés ; et
une couche externe d'un polymère filmogène entourant ladite couche de matrice formant barrière, la couche externe étant sensiblement dépourvue de pigments de telle manière à former un revêtement transparent.

2. Granule selon la revendication 1, dans lequel la couche de matrice formant barrière comprend en outre un agent tensioactif ou un agent mouillant.

3. Granule selon la revendication 1, dans lequel ledit agent desséchant est constitué de particules sensiblement insolubles dans l'eau et dispersibles dans l'eau ayant un diamètre moyen ne dépassant pas 30 microns environ.

4. Granule selon la revendication 1, dans lequel ledit agent desséchant est constitué au moins en partie de pigment ou d'argile.

5. Granule selon la revendication 4, dans lequel ledit agent desséchant est choisi dans le groupe consistant en TiO₂, talc, carbonate de calcium, bentonite, terre à diatomées, et l'un quelconque de leurs mélanges.

6. Granule selon la revendication 1, dans lequel le polysaccharide est choisi dans le groupe consistant en amidon, caragénine, cellulose, gomme arabique, gomme d'acacia, gomme de xanthorrée, gomme de caroube et gomme guar, et l'un quelconque de leurs mélanges.

7. Granule selon la revendication 1, dans lequel le sucre ou l'alcool de sucre est choisi dans le groupe consistant en glucose, fructose, raffinose, maltose, lactose, tréhalose et saccharose, mannitol, sorbitol, inositol, et l'un quelconque de leurs mélanges.

8. Granule selon la revendication 2, dans lequel ledit agent tensioactif ou agent mouillant est le Neodol.

9. Granule selon la revendication 1, dans lequel la protéine est une enzyme choisie dans le groupe consistant en protéase, amylase, lipase et cellulase.

10. Procédé de préparation d'un granule selon la revendication 1, comprenant :
(i) la fourniture d'une particule contenant des protéines,
(ii) la formation d'une couche de matrice formant barrière autour de la particule par application d'un mélange qui comprend un agent desséchant, un polysaccharide et un sucre ou un alcool de sucre finement divisés, et
(iii) l'application d'un revêtement transparent d'un polymère filmogène autour de ladite couche de matrice formant barrière, la couche externe étant sensiblement dépourvue de pigments.
